# EUROPEAN PATENT SPECIFICATION

(11) **EP 1 014 855 B1**
(45) Date of publication and mention of the grant of the patent: **19.07.2006**
(21) Application number: 98919344.6
(22) Date of filing: 29.04.1998
(51) Int. Cl.: A61B 5/087

(54) **PEAK FLOW MONITOR**
SPITZEN-DURCHFLUSSMESSER
DISPOSITIF SERVANT A CONTROLER UN DEBIT DE POINTE

(30) Priority: 29.04.1997 ZA 9703679
(43) Date of publication of application: 05.07.2000
(73) Proprietor: Gassner, Martin, Loevenstein, 7530 (ZA); Wessels, Jacobus, Loevenstein, 7530 (ZA)
(72) Inventor: FOURIE, Pieter, Rousseau, Western Cape (ZA)
(74) Representative: Gemmell, Peter Alan
(86) International application number: PCT/GB1998/001253
(87) International publication number: WO 1998/048694

(56) References cited:
- WO-A-96/30076
- WO-A-96/37147
- WO-A-98/02206

## Description

### Background to the invention

This invention relates to a peak expiratory flow monitoring device.

Peak expiratory flow rate (PEFR) is a measure of lung function which can be easily and accurately determined by several devices. The monitoring of PEFR has gained acceptance as a means of monitoring the state of asthma, since it can be recorded reliably by cooperative subjects without the help of technically skilled personnel and with the use of simple, portable devices.

A number of these devices are described in WO-A-96/37147, which discloses, in particular, a device according to the preamble of claim 1.

### Summary of the invention

According to this invention a flow monitoring device comprises a substantially hollow body formed with a fluid flow passage incorporating a signal generator adapted to generate a signal in dependence on the achievement of a predetermined volume flow rate of fluid across the signal generator, the body comprising first and second body shells that are rotatably interengageable with one another to define a resonance chamber formed with a vent outlet, the first body shell being formed with an inlet constituted by a mouthpiece and the fluid flow passage extending at least between the mouthpiece and the vent outlet, the device including an occluder that is adapted to occlude the vent outlet to a greater or lesser extent depending on the rotational position of the body shells relatively to one another and the body shells including a locking mechanism by means of which the body shells may be locked to one another in increments to set the occluder at a predetermined incremental variation of the vent outlet size, characterised in that the locking mechanism comprises multiple splines formed on one of the body shells and a key movably mounted on the other body shell, the key being adapted for axial movement into the grooves between the splines to lock the body shells to one another.

In the preferred form of the invention, the resonance chamber, with the outlet formed therein, may be defined largely by the second body shell and the occluder may be incorporated in the first body shell and adapted, when the body shells are interengaged, to extend into the resonance chamber to occlude the vent outlet to a greater or lesser extent depending on the rotational position of the body shells relatively to one another. In this form of the invention, the first body shell can be referred to as an inlet shell and the second body shell can be referred to as an outlet shell.

The body shell with the key is preferably formed with a slideway with the key being mounted on a key carrier that is slidably mounted in the slideway.

In this form of the invention, the key carrier may include a locking pin that is adapted for insertion and retention in a complemental locking aperture formed in the body shell.

The occluder is preferably cylindrical and adapted, when the shells are interengaged, to extend into the outlet shell, the inwardly extending edge of the occluder being spirally shaped- and- adapted to occlude the vent to a greater or lesser extent depending on the rotational position of the shells relatively to one another.

The body shells may be interconnected by the provision, in one of the body shells, of an internal axial spindle and, on the other body shell, a spindle mount within which the spindle may be rotatably mounted. The spindle and mount are preferably interengaged such that axial disengagement of the spindle and mount are prevented other than by destruction of the monitoring device.

### Brief description of the drawings

In the drawings:
Figure 1 an exploded perspective view of the monitoring device according to the invention, seen from the outlet end of the device;
Figure 2 is a perspective view of the device of figure 1, seen from the inlet end of the device;
Figure 3 is a diagrammatic section on a line 3-3 in figure 1;
Figure 4 is a diagrammatic section on a line 4-4 in figure 2;
Figure 5 is an end elevation (partly in section) of the device of the invention, seen from the outlet end thereof (without a sound generator);
Figure 6 is a side elevation of the outlet body shell of the device of figure 1;
Figure 7 is an end elevation of a part of a locking mechanism of the device of figure 1;
Figure 8 is a plan view on the part of the locking mechanism illustrated in figure 7; and
Figure 9 is a partial under plan view on the part of the locking mechanism illustrated in figure 7.

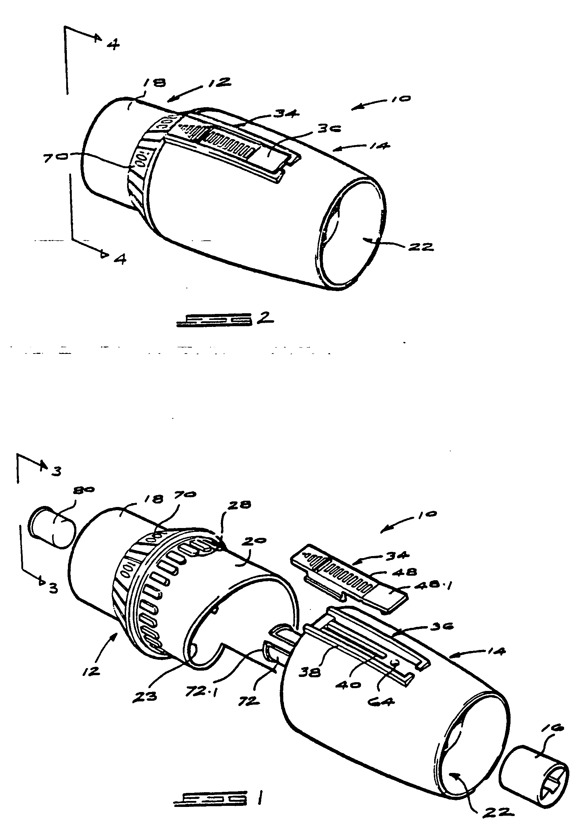

### Description of embodiments of the invention

The monitoring device of the invention is not a flow meter in the sense that it does not measure absolute flow through the device. Instead, it is a device intended as a threshold monitor of the peak expiratory flow rate (PEFR) of a subject. This is done by monitoring the achievement or otherwise of a predetermined threshold PEFR by the subject. In this sense, the monitor of this invention is a threshold monitor in that it monitors the subject's achieved PEFR as compared to a diagnostic threshold.

The predetermined PEFR is a diagnostic threshold that will depend on the treatment protocol prescribed for the subject after measurement of the subject's anticipated or predicted PEFR.

The monitor 10 of the invention can be seen to comprise a pair of interengageable body shells 12, 14 and a sound generator constituted by a reed assembly 16.

The shell 12 is essentially an inlet shell and the shell 14 is an outlet shell that is joined together in the manner described below.

In practice, the subject breathes in as far as possible and then blows out or expires through the cylindrical tube constituting the mouthpiece 18 at the inlet end of the inlet shell 12. The subject is normally instructed not to cough nor to allow explosive expiratory flow, such as spitting, coughing or interrupted flow resulting from tongue obstructions of the mouthpiece, but as will be seen below, the monitoring device 10 of the invention is designed to minimise the possible inaccuracies arising from such explosive expiratory flow.

The outlet shell 14 is constituted by an external wall 24 that encloses a resonance chamber 46 formed with a vent opening 26 that extends axially long the length of the outlet shell 14. A horn-shaped central housing 22 for the reed assembly 16 extends axially within the resonance chamber 46.

The shells 12, 14 are intended for interconnection with one another and the reed assembly 16 is pressed into the horn-shaped housing 22.

The mouthpiece 18 extends into a cylindrical occluder 20, the upper edge 23 of which is spirally cut. When the shells 12, 14 are joined, the occluder 20 extends into the outlet shell 14 to occlude the vent 26 to a greater or lesser extent depending on the location of the upper edge of the occluder 20 relatively to the walls of vent 26, which depends, in turn, on the degree of rotation of the shells 12, 14 relatively to one another.

When the subject blows through the mouthpiece 18 and the outlet shell 14, a portion of the subject's expired flow will escape through the vent outlet 26 and a portion thereof will be directed through the reed assembly 16. By rotating the inlet shell 12 relatively to the outlet shell 14, the spiral occluder 20 occludes the vent opening 26 to a greater or lesser extent thereby determining the extent to which expired flow will be directed through the reed assembly 16. If sufficient flow is directed through the reed assembly 16, it will generate sound.

The reed assembly 16 is nonlinear (a linear whistle being one that will produce with reasonable accuracy double the sound intensity for doubling of the flow rate), and the length, shape and mass of the reed are chosen so as to produce a sound within the frequency range that human hearing is most sensitive. The reed is adapted to produce sound with a pitch of approximately 1000Hz. While the reed is nonlinear, the device 10 can, in effect, be "linearised" by proper design of the spiral occluder.

More important, the reed assembly 16 provides a clear and rapid onset of sound when its flow threshold is exceeded. In this regard it can be viewed as an analogue-to-digital device which is either "on" or "off" with nothing in between. To this end, the reed assembly 16 is manufactured to exacting tolerances to ensure minimum variation from unit to unit so that each reed is activated at the same flow threshold. This eliminates the need to calibrate every monitoring device 10.
In addition, the reed is shaped to ensure that the dynamic and static flow thresholds of the reed assembly 16 are close to one another to minimise erroneous sound generation due to large fluctuations in the flow, such as might occur during explosive as opposed to smooth expiratory flow through the device 10.

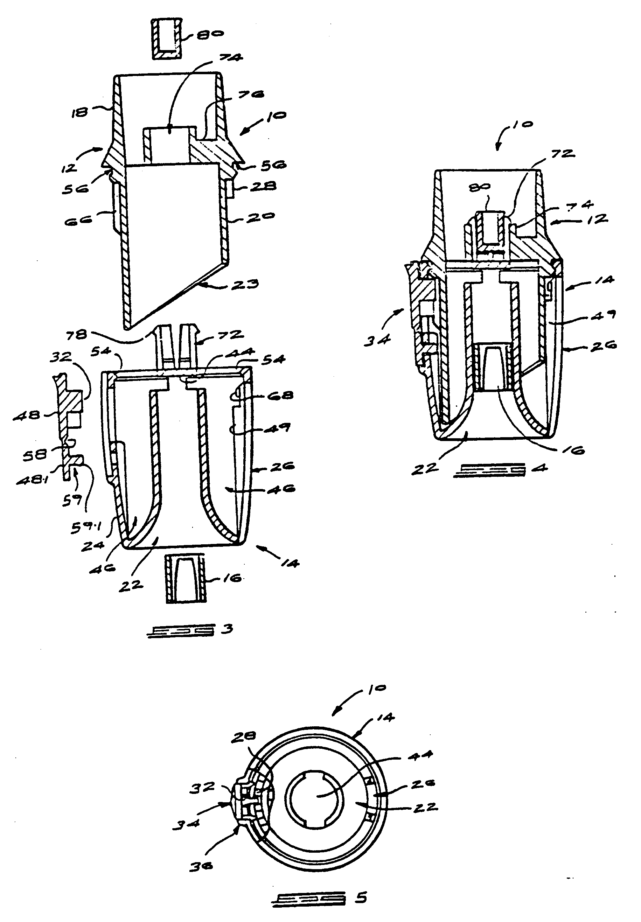

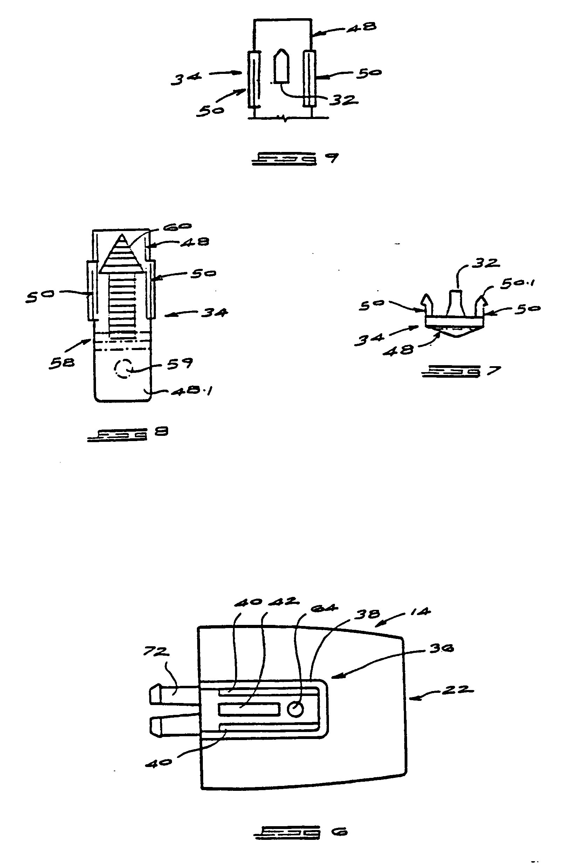

Referring particularly to figure 4, the reed assembly 16 is pressed into the housing 22 extending down the interior of the outlet shell 14, the outer shape of the reed assembly 16 and the inner shape of the housing 22 being complementally frusto-conical. The inlet end of the reed assembly 16 is open to the interior of the inlet end of the housing 22 and the outlet end of the reed assembly opens outwardly to atmosphere within the horn-shaped mouth of the housing 22. The horn-shaped mouth enhances the audibility of the device 10 as does the outlet shell 14 which forms a resonance cavity 46 around the housing 22.

The inlet end of the housing 22 is formed with a transversely extending barrier 44. The barrier 44 acts as a low pass filter and assists in preventing the reed in the reed assembly 16 from being activated by the subject using explosive or impulse-like flow techniques in order to obtain an overly optimistic result in a test.

The barrier 44 is constituted by a barrier plate located at the inlet end of the horn-shaped outlet 22. As can be seen from Figure 5, the outer diameter of the barrier plate 44 is slightly less than the inner diameter of the outlet horn 22 at its inlet end to facilitate the passage of expiratory flow linked to the outlet horn 22.

The inlet and outlet shells 12, 14 are formed with complementary engagement formations by means of which the two parts 12, 14 can be clipped together.

One of these engagement formations is integral with the barrier 44. On the side facing the mouthpiece 18, the barrier 44 is provided with a tubular spindle 72 that is dimensioned for rotatable location within a hub 74 formed within in the mouthpiece 18. The hub 74 is carried on three radially extending arms 76.

The spindle 72 is easily rotatable within in the hub 74 and it is retained in position by means of an outwardly extending flange 78 that constitutes a clip formation. When the spindle 72 is in position in the hub 74, the flange projects beyond the rim of the hub 74. The spindle 72 is split in three places to facilitate insertion thereof into the hub 74.

To prevent removal of the spindle 72 from the hub 74, a locking lug 80 is pressed into the tubular interior of the spindle 72. The locking lug 80 is dimensioned not to splay the spindle 72, but merely to prevent the removal of the spindle 78 to the hub 74 without destruction of the monitor 10.

In addition to the spindle 72 and hub 74, the engagement formations include an inwardly directed bead 54 formed on the engagement end of the external wall 24 of the outlet shell 14 and an outwardly directed groove 56 formed to extend peripherally about the exterior of the tubular occluder 20. The bead 54 and the engagement end of the inlet shell 14 as well as the groove 56 and the occluder 20 are dimensioned for the inlet and outlet shells 12, 14 to clip together with the bead 54 engaging slidingly within the groove 56. The bead 54 and the groove 56 are smoothly finished to ensure easy rotational movement of the body shells 12,14.

The edges of the vent aperture 26 are bounded by inwardly directed blanking walls 49 connected to the outer wall 24 of the outlet shell 14. The blanking walls 49 extend into the resonance chamber 46 and are dimensioned to slide closely over the outer surface of the occluder 20, thereby to blank off the gap between the occluder and the outer wall 24 of the outlet shell resonance chamber 46.

The base of the cylindrical wall of the occluder 20 is provided with multiple splines 28 that are evenly distributed about the circumference of the occluder 20. The splines 28 form part of the locking mechanism of the monitoring device 10 of the invention.

The locking mechanism includes a key 32 that is adapted for axial insertion into the grooves between the splines 28.

The key 32 is integral with a key carrier 34 that, in use, is mounted in a slideway 36 formed in the outlet shell 14.

This locking mechanism is intended to lock the body shells 12, 14, against relative rotation. The locking mechanism is intended to be semi permanent so that, once locked in place, the locking mechanism cannot be undone without destroying the monitor 10. At the very least, the locking mechanism must be difficult to "unlock".

In the monitor 10 of the invention, the locking mechanism is made difficult to undo by the structure of the slideway 36 and the key carrier 34.

As can be seen from Figure 6, the slideway 36 is bounded by an external wall formation 38 and includes a pair of parallel slots 40 formed, in the wall 24 of the chamber 46 on either side of a central slot 42 formed in the wall 24 of the chamber 46.

The key carrier 34 is illustrated in greater detail in Figures 7, 8 and 9. In these drawings, the key carrier 34 can be seen to comprise a slider plate 48 from the underside of which the key 32 and a pair of slider clips 50 depend. The slider clips 50 clip into the slots 40 of the slideway to retain the key carrier 34 within the slideway 36 while the key 32 fits into the central slot 42 in the slideway 36.

By locking the inlet shell 12 to the outlet shell 14 in a predetermined rotational position, the spiral occluder 20 is locked in a particular position relatively to the vent 26, thereby to determine the effective vent opening. As has been stated above, the vent opening will be determined by the treatment protocol.

The splines 28 permit the adjustment of the monitoring device 10 in small increments.

The occluder 20 is provided with a stop formation 66 that is positioned to catch against corresponding stops 68 at the maximum rotational positions of the inlet shell 12 relatively to the outlet shell 14.

The stop formation 64 will butt up against the stops 68 in either the maximum "closed" position or the maximum "open" position of the monitoring device 10.

In the maximum "closed" position, the upper surface 23 of the occluder is as close to the upper extremity of the vent 26 as rotation of the body shells 12, 14 will allow. At the other rotational extremity In the maximum "open" position, the upper surface 23 of the occluder is as close to the lower extremity of the vent 26 as relative rotation of the shells 12, 14 will allow.

In use, the practitioner will determine the subject's diagnostic threshold whereupon the clinician will assemble the device 10 or instruct the subject in the assembly of the device 10, by engagement of the inlet and outlet shells 10, 12, to one another with the occluder 20 located in the position determined by the subject's treatment protocol. To assist in such assembly, appropriate markings 70 are made on the mouthpiece 18. The appropriate markings 70 are matched up in accordance with the treatment protocol. The markings 70 take the form of flow rate values.

The subject is required merely to implement the home monitoring regime - the subject will be told when flow should be measured and how the monitoring device is to be used.

The slider clips 50 include clip formations 50.1 that retain the key carrier 34 within the slideway 36, but the slider clips 50 and the key 32 as well as the slots 40, 42 are dimensioned to permit relatively easy sliding of the key carrier 34 within the slideway 36.

The slider plate 48 of the key carrier 34 is formed with a V-shaped line of weakness 58 on the underside thereof to provide a locking tab 48.1. The line of weakness 58 permits easy bending of the locking tab 48.1 out of the plane of the slider plate 48. In this position, the bent locking tab 48.1 permits easy assembly of the locking mechanism, since it permits the location of the key carrier 34 in the slideway 36 with the key 32 positioned remotely of the splines 28 formed on the inlet body shell 12.

The locking tab 48.1 of the slider plate 48 is provided with a downwardly depending locking pin 59. The purpose of the pin 59 is to lock the key carrier 34 in position within the slideway 36 after setting of the correct rotational positions of the body shells 12, 14.

This is done by sliding the key carrier 34 in the direction of the mouthpiece 18. An arrow 60 is moulded onto the upper surface of the slider plate 48 in order to clarify the desired sliding movement.

Before the key carrier 34 is slid into place as described above, the practitioner selects the correct rotational setting of the body shells 12, 14 relatively to one another in accordance with the treatment prescribed for the subject. With the correct rotational position selected, the key carrier 34 is slid home so that the key 32 engages the appropriate groove between the splines 28 formed on the occluder 20 on the inlet body shell 12. The facing ends of the splines 28 are rounded and the leading end of the key 32 is tapered, both of which facilitate the entry of the key 32 into the grooves between the splines 28.

With the key 32 in position between the splines 28, the locking tab 48.1 of the slider plate 48 can be bent back into the plane of the slider plate 48.

To do this, the locking pin 59 is forced into a socket. 64 formed in the slideway 36.

The locking pin 59 is formed with an enlarged head 59.1 which requires some force to permit ingress of the head into the socket 64.

With the locking pin 59 in place in the socket 64, the locking tab 48.1 of the slider plate 48 is retained within in the slideway walls 38. The gap between the slider plate 48 and the slideway walls 38 is made as small as possible. This ensures that it is difficult to obtain any purchase on the slider plate if any attempt is made to "unlock" the locking mechanism, for instance, by attempting to raise the locking tab 48.1 against the retention of the locking pin 59 in the socket 64.

If required, the device 10 can be-predesigned for use by children or adults. In the former case, the size of the vent 26 can be predetermined to permit peak flows of up to 450l.min⁻¹, while in the latter case, the size of the vent 26 can be predetermined to permit peak flows of up to 8001.min-1. The device could conveniently be calibrated for adult use and, alternatively or in addition, one or more additional vent outlets (not shown) can be formed in the resonance chamber 46, preferably in the wall thereof constituted by the horn-shaped outlet 22.

## Claims

1. A flow monitoring device comprising a substantially hollow body formed with a fluid flow passage incorporating a signal generator (16) adapted to generate a signal in dependence on the achievement of a predetermined volume flow rate of fluid across the signal generator (16), the body comprising first and second body shells (12, 14) that are rotatably interengageable with one another to define a resonance chamber (46) formed with a vent outlet (26), the first body shell (12) being formed with an inlet constituted by a mouthpiece (18) and the fluid flow passage extending at least between the mouthpiece (18) and the vent outlet (26), the device including an occluder (20) that is adapted to occlude the vent outlet (26) to a greater or lesser extent depending on the rotational position of the body shells (12, 14) relatively to one another and the body shells (12, 14) including a locking mechanism by means of which the body shells (12, 14) may be locked to one another in increments to set the occluder (20) at a predetermined incremental variation of the vent outlet size, **characterised in that** the locking mechanism comprises multiple splines (28) formed on one of the body shells and a key (32) movably mounted on the other body shell, the key (32) being adapted for axial movement into the grooves between the splines to lock the body shells (12, 14) to one another.

2. A flow monitoring device according to claim 1 in which the resonance chamber (46), with the outlet (26) formed therein, is defined largely by the second body shell (14) and the occluder (20) is incorporated in the first body shell (12) and adapted, when the body shells (12, 14) are interengaged, to extend into the resonance chamber (46) to occlude the vent outlet (26) to a greater or lesser extent depending on the rotational position of the body shells (12, 14) relatively to one another.

3. A flow monitoring device according to either of the preceding claims in which the body shell with the key is formed with a slideway (36) and the key is mounted on a key carrier (34) that is slidably mounted in the slideway (36).

4. A flow monitoring device according to claim 3 in which the key carrier (34) includes a locking pin (59) that is adapted for insertion and retention in a complemental locking aperture (64) formed in the body shell.

5. A flow monitoring device according to any one of the preceding claims, the occluder (20) of which is cylindrical and adapted, when the body shells (12, 14) are interengaged, to extend into the second body shell, the inwardly extending edge of the occluder (20) being spirally shaped and adapted to occlude the vent (26) to a greater or lesser extent depending on the rotational position of the body shells (12, 14) relatively to one another.

6. A flow monitoring device according to any one of the preceding claims in which the body shells (12, 14) are interconnected by the provision, in one of the body shells, of an internal axial spindle (72) and, on the other body shell, a spindle mount (74) within which the spindle (72) may be rotatably mounted.

7. A flow monitoring device according to claim 6 in which the spindle (72) and mount (74) are adapted for interengagement such that axial disengagement of the spindle (72) and mount (74) are prevented other than by destruction of the monitoring device.

## Patentansprüche

1. Durchflunsüberwachungsvorrichtung, die einen im Wesentlichen hohlen Körper umfasst, der mit einem Fluidflussdurchgang ausgebildet ist, der einen Signalerzeuger (16) enthält, welcher zur Erzeugung eines Signals in Abhängigkeit vom Erreichen eines vorbestimmten Völumenflidduchflusses über den Signalerzeuger (16) ausgeführt ist, wobei der Körper ein erstes und ein zweites Körpergehäuse (12, 14) umfasst, die drehbar miteinander in Eingriff gebracht werden können, um eine Resonanzkammer (46) zu definieren, die mit einem Lüftungsauslass (26) ausgebildet ist, wobei das erste Körpergehäuse (12) mit einem durch ein Mundstück (18) gebildeten Einlass ausgebildet ist und sich der Fluidflussdurchgang mindestens zwischen dem Mundstück (18) und dem Lüftungsauslass (26) erstreckt, wobei die Vorrichtung ein Verschlusselement (20) enthält, das dazu ausgeführt ist, den Lüftungsauslass (26) in Abhängigkeit von der Drehstellung der Körpergehäuse (12, 14) bezüglich einander mehr oder weniger zu verschließen, wobei die Körpergehäuse (12, 14) einen Verriegelungsmechanismus enthalten, mittels dessen die Körpergehäuse (12, 14) in Inkrementen miteinander verriegelt werden können, um das Verschlusselement (20) bei einer vorbestimmten inkrementalen Änderung der Lüftungsauslassgröße einzustellen, **dadurch gekennzeichnet, dass** der verriegelungsmechanismus mehrere an einem der Körpergehause ausgebildete Keilverzehnungen (28) und einen am anderen Kötpexgehäuse beweglich angebrachten Keil (32) umfasst, wobei der Keil (32) zur Axialbewegung in die Nuten zwischen den Keilverzahnungen zwecks Verriegelung der Körpergehäuse (12, 14) miteinander ausgeführt ist.

2. Durchflussuberwachungsvorrichtung nach Anspruch 1, bei der die Resonanzkammer (46) mit dem darin ausgebildeten Auslass (26) weitgehend durch das zweite Körpergehäuse (14) definiert wird und das Verschlusselement (20) in dem ersten Körpergehäuse (12) enthalten und dazu ausgeführt ist, bei Eingriff der Körpergehäuse (12, 14) miteinander in die Resonanzkammer (46) zu ragen, um den Lüftungsauslass (26) in Abhängigkeit von der Drehstellung der Körpergehäuse (12, 14) bezüglich einander zu einem größeren oder geringeren Ausmaß zu verschließen.

3. Durchflussüberwachungsvorrichtung nach einem der vorhergehenden Ansprüche, bei der das Körpergehäuse mit dem Keil mit einer Führungsbahn (36) ausgebildet und der Keil an einem verschiebbar in der Führungsbahn (36) angebrachten Keilträger (34) angebracht ist.

4. Durchflussüberwachungsvorrichtung nach Anspruch 3, bei der der Keiltrager (34) einen Verriegelungsstift (59) enthält, der zum Einführen und Halten in einer im Körpergehäuse ausgebildeten komplementären Verriegelungsöffnung (64) ausgeführt ist.

5. Durchflussüberwachungsvorrichtung nach einem der vorhergehenden Ansprüche, deren Verschlusselement (20) zylindrisch und dazu ausgeführt ist, bei Eingriff der Körpergehäuse (12, 14) miteinander in das zweite Körpergehäuse zu ragen, wobei der nach innen ragende Rand des Verschlusselements (20) spiralförmig ausgebildet und dazu ausgeführt ist, die Lüftung (26) in Abhängigkeit von der Drehstellung der Körpergehäuse (12, 14) bezüglich einander in einem größeren oder geringeren Ausmaß zu verszhließen.

6. Durchflussüberwachungsvorrichtung nach einem der vorhergehenden Ansprüche, bei der die Körpergehäuse (12, 14) durch Vorsehen einer inneren Axialspindel (72) in einem der Körpergehäuse und einer Spindelhalterung (74) am anderen Körpergehäuse, in der die Spindel (72) drehbar angebracht werden kann, miteinander verbunden sind.

7. Durchflussüberwachungsvorrichtung nach Anspruch 6, bei der die Spindel (72) und die Halterung (74) zum Eingriff miteinander ausgeführt sind, so dass ein axiales Ausrücken der Spindel (72) und der Halterung (74) mit Ausnahme von Zerstörung der Überwachungsvorrichtung verhindert wird.

## Revendications

1. Dispositif de contrôle d'écoulement comprenant un corps sensiblement creux formé avec un passage d'écoulement de fluide comprenant un générateur de signal (16) agencé pour générer un signal en fonction de l'obtention d'un débit volumique de fluide prédéterminé à travers le générateur de signal (16), le corps comprenant des première et deuxième enveloppes de corps (12, 14) qui sont aptes à s'engager par rotation l'une avec l'autre pour définir une chambre de résonance (46) formée avec un orifice d'évent (26), la première enveloppe de corps (12) étant formée avec une entrée constituée par une embouchure (18) et le passage d'écoulement de fluide s'étendant au moins entre l'embouchure (18) et l'orifice d'évent (26), le dispositif comprenant un dispositif de fermeture (20) qui est adapté pour fermer l'orifice d'évent (26) dans une mesure plus ou moins grande en fonction de la position en rotation des enveloppes de corps (12, 14) l'une par rapport à l'autre et les enveloppes de corps (12, 14) comprenant un mécanisme de verrouillage au moyen duquel les enveloppes de corps (12, 14) peuvent être verrouillées l'une à l'autre selon des incréments pour régler le dispositif de fermeture (20) selon une variation à incrément prédéterminée de la dimension de l'orifice d'évent, **caractérisé en ce que** le mécanisme de verrouillage comprend des cannelures multiples (28) formées sur l'une des enveloppes de corps et une clavette (32) montée de façon mobile sur l'autre enveloppe de corps, la clavette (32) étant agencée pour un mouvement axial dans les gorges entre les cannelures pour verrouiller les enveloppes de corps (12, 14) l'une à l'autre.

2. Dispositif de contrôle d'écoulement selon la revendication 1, dans lequel la chambre de résonance (46) avec l'orifice (26) formé dans celle-ci est définie largement par la deuxième enveloppe de corps (14) et le dispositif de fermeture (20) est incorporé dans la première enveloppe de corps (12) et adapté, lorsque les enveloppes de corps (12, 14) sont engagées mutuellement, pour s'étendre dans la chambre de résonance (46) pour fermer l'orifice d'évent (26) selon une mesure plus ou moins grande en fonction de la position en rotation des enveloppes de corps (12, 14) l'une par rapport à l'autre.

3. Dispositif de contrôle d'écoulement selon l'une quelconque des revendications précédentes, dans lequel l'enveloppe de corps avec la clavette est formée avec une glissière (36) et la clavette est montée sur un support de clavette (34) qui est monté coulissant dans la glissière (36).

4. Dispositif de contrôle d'écoulement selon la revendication 3, dans lequel le support de clavette (34) comprend une broche de verrouillage (59) qui est adaptée pour être insérée et retenue dans une ouverture de verrouillage complémentaire (64) formée dans l'enveloppe de corps.

5. Dispositif de contrôle d'écoulement selon l'une quelconque des revendications précédentes, dans lequel le dispositif de fermeture (20) est cylindrique et adapté, lorsque les enveloppes de corps (12, 14) sont engagées mutuellement pour s'étendre dans la deuxième enveloppe de corps, le bord s'étendant vers l'intérieur du dispositif de fermeture (20) étant en forme de spirale et adapté pour fermer l'évent (26) dans une mesure plus ou moins grande en fonction de la position en rotation des enveloppes de corps (12, 14) l'une par rapport à l'autre.

6. Dispositif de contrôle d'écoulement selon l'une quelconque des revendications précédentes, dans lequel les enveloppes de corps (12, 14) sont reliées entre elles par la fourniture, dans l'une des enveloppes de corps, d'une broche axiale interne (72) et, sur l'autre enveloppe de corps, d'une monture de broche (74) dans laquelle la broche (72) peut être montée en rotation.

7. Dispositif de contrôle d'écoulement selon la revendication 6, dans lequel la broche (72) et la monture (74) sont adaptées pour s'engager mutuellement de sorte que le désengagement axial de la broche (72) et de la monture (74) est empêché d'une façon autre que par une destruction du dispositif de contrôle.
